# EUROPÄISCHE PATENTSCHRIFT

(11) **EP 2 757 937 B1**
(45) Veröffentlichungstag und Bekanntmachung des Hinweises auf die Patenterteilung: **05.06.2019**
(21) Anmeldenummer: 12775611.2
(22) Anmeldetag: 20.09.2012
(51) Int. Cl.: A61B 3/103, A61B 3/117, A61B 3/13, A61B 3/00, A61F 2/16

(54) **OPTIKANORDNUNG UND VERFAHREN ZUM ERMITTELN DER AUSRICHTUNG EINER KÜNSTLICHEN LINSE**
OPTICAL ARRANGEMENT AND METHOD FOR ASCERTAINING THE ORIENTATION OF AN ARTIFICIAL LENS
SYSTÈME OPTIQUE ET PROCÉDÉ PERMETTANT DE DÉTERMINER L'ORIENTATION D'UN CRISTALLIN ARTIFICIEL

(30) Priorität: 23.09.2011 DE 102011114251
(43) Veröffentlichungstag der Anmeldung: 30.07.2014
(73) Patentinhaber: Vossamed GmbH & Co. KG, 50667 Köln (DE)
(72) Erfinder: GERTEN, Georg, 53121 Bonn (DE)
(74) Vertreter: Grünecker Patent- und Rechtsanwälte PartG mbB
(86) Internationale Anmeldenummer: PCT/EP2012/003946
(87) Internationale Veröffentlichungsnummer: WO 2013/041230

(56) Entgegenhaltungen:
- WO-A1-2011/030509
- DE-A1- 1 547 417
- DE-A1- 10 017 298
- DE-A1-102009 052 128
- DE-C1- 4 316 782
- US-A1- 2002 154 269

## Beschreibung

Die Erfindung bezieht sich auf eine Optikanordnung und auf ein Verfahren zum Ermitteln der Ausrichtung einer künstlichen Linse in einem Auge. Solche künstlichen Linsen werden auch als Intraokularlinsen (IOL) bezeichnet. Ihre Ausrichtung, insbesondere ihre Rotationsausrichtung, ist vor allem bei so genannten torischen Intraokularlinsen von Interesse, die in zwei unterschiedlichen Hauptachsen zwei verschiedene Krümmungsradien und damit zwei verschiedene Brechkräfte haben.

Operationen an der menschlichen Augenlinse gehören zu den weltweit am häufigsten durchgeführten Operationen. Solche Operationen werden notwendig durch pathologische Prozesse, die meist das Innere der Augenlinse betreffen, beispielsweise durch eine Trübung der Augenlinse (Kataraktbildung) und/oder eine Verhärtung des Linsenkernes bei der Alterssichtigkeit. Mit den modernen Operationsverfahren wird das Innere der Augenlinse (Kern, Rinde) entfernt, wozu der Linsenkapselsack möglichst nur von vorne eröffnet und im Übrigen stehengelassen wird. Der nach dem Entfernen des Inneren der natürlichen Augenlinse dann leere Kapselsack dient dann im Allgemeinen zur Aufnahme einer künstlichen Intraokularlinse (IOL), die die entfernten inneren Teile der Augenlinse ersetzt und die Sehfähigkeit des Patienten unter Berücksichtigung der gewünschten optischen Korrektur des Auges wieder herstellt.

Moderne Intraokularlinsen können individuell für den betroffenen Patienten hergestellt werden und hierzu die unterschiedlichsten optischen Eigenschaften im Hinblick auf Asphärizität, Multifokalität, torische Oberflächen u.dgl. aufweisen, um den verschiedenen Ursachen der (natürlichen) Fehlsichtigkeit des betroffenen Patienten gerecht zu werden und diese zu korrigieren. Damit diese der individuell hergestellten Linse eigenen Merkmale ihre Funktion möglichst korrekt erfüllen, wird eine möglichst optimale Zentrierung der Linse und ihre Zuordnung zu bestimmten Bezugssystemen im optischen System des Auges angestrebt. So soll im Allgemeinen die Intraokularlinse konzentrisch zur Pupillenmitte unter Berücksichtigung der Gesichtslinie und Mitte der Eintrittspupille, insbesondere im Kapselsack, aber auch im Sulcus Ciliaris oder der Vorderkammer des Auges zuverlässig platziert werden, wobei speziell bei torischen Intraokularlinsen bzw. phaken torischen IOL (IOL, die zusätzlich zur eigenen Linse implantiert werden) zusätzlich eine achsgenaue Positionierung wichtig ist. Dabei wird das vorhergesagte Refraktionsziel vom tatsächlich Erreichten bei einer Fehlrotation um so mehr abweichen, je größer die Rotation und die Brechkraft des Zylinderanteils der implantierten Linse sind. Es kommt aber nicht nur auf die korrekte Ausrichtung in X-und Y-Achse des optischen Systems des Auges an, sondern es ist auch eine möglichst dauerhafte und genaue Positionierung der Linse in Z-Richtung (entlang der optischen Achse) ohne Verkippung der IOL wünschenswert.

Zwar gibt es Mess- und Projektionssysteme, die dem Operateur während der Operation die entsprechende Refraktion und die sich hieraus ergebenden Achsen bzw. über bestimmte Bezugspunkte die Achsen im Auge anzeigen können. Diese optischen Bestimmungssysteme berechnen die Stärke der IOL aus der Gesamtbrechkraft des Auges.

Es ist jedoch eine direkte Messung der optischen Stärke und Position der Linse über ihre Krümmungsradien wünschenswert. Speziell wenn ein Astigmatismus des Auges, meist ausgehend von der Cornea (sogenannte Hornhaut-Verkrümmung) mit einer IOL korrigiert werden soll, muss auf eine exakte Übereinstimmung der zu korrigierenden Achse des cornealen Astigmatismus und der Zylinderachse der torischen IOL geachtet werden. Schon bei kleiner Fehlrotation der IOL Achse entstehen schnell größere Fehler des gesamten Abbildungssystems Auge, so bedingen ca. 15° Fehlrotation einer IOL bereits ca. 50% Verlust an Zylinder-Korrektur und eine erhebliche Achsrotation des verbliebenen Gesamtfehlers im optischen System.

Bei torischen IOL wird die Achse der Zylinderwirkung in der Regel durch Hersteller-Markierungen auf der Optik angegeben, wie beispielsweise in der WO 2011/030509 A1 beschrieben. Diese haben jedoch eine gewisse Fehlertoleranz von üblicherweise 3°, die zu entsprechenden Gesamtfehlern in der Refraktion des Auges führen können (siehe oben).

Aus der US 2002/0154269 A1 geht eine Vorrichtung zur stereoskopischen Untersuchung eines Auges hervor, bei der mehrere Beobachtungsstrahlengänge vorgesehen sind, die zwingend auf unterschiedliche Optiken abgebildet werden.

Bei einem aus der DE 15 47 417 A1 bekannten Verfahren erfolgt das Einstrahlen eines leuchtenden Objekts ausschließlich axial zum Messstrahlengang.

Ophtalmologische Geräte und Verfahren zum Überprüfen der optischen Eigenschaften eines Auges mit oder ohne künstliche Linse sind ferner bekannt beispielsweise aus der DE 10 2008 034 490 A1, der DE 10 2005 042 436 A1, der DE 10 2005 031 496 A1, der DE 295 17 578 U1, der DE 198 17 047 A1, der JP 01308552A, der CH 699 886 A1, der WO 2011/030509 A1, der CA 1099965 oder der DE 26 43 344 A1. Es hat sich jedoch herausgestellt, dass diese Geräte entweder kompliziert in ihrer Handhabung oder nicht dazu geeignet sind, zuverlässig die Ausrichtung einer künstlichen Linse in einem Auge zu bestimmen.

Hier soll nun die Erfindung Abhilfe schaffen, die es sich zur Aufgabe gemacht hat, dass eine Intraokularlinse möglichst präzise in den Kapselsack, den Sulcus Ciliaris oder auch die Vorderkammer eines Auges eingesetzt und nach korrekter Ausrichtung in die gewünschte Position in dieser Lage zuverlässig festgelegt werden kann.

Diese Aufgabe wird gelöst durch eine Optikanordnung mit den Merkmalen des Anspruchs 1 beziehungsweise durch ein Verfahren mit den Merkmalen des Anspruchs 14. Vorteilhafte Weiterbildungen in den Erfindung sind in den Unteransprüchen angegeben.

Die erfindungsgemäße Optikanordnung verfügt über mindestens zwei Licht ausstrahlende Testobjekte mit definierter Kontur. Über eine Kollimationsoptik wird das von diesen Testobjekten ausgehende strukturierte Lichtmuster kollimiert und auf ein Auge gerichtet, das über einen Beobachtungsstrahlengang der Optikanordnung von einem Bediener der Optikanordnung oder beispielsweise auch einer Kamera beobachtet wird. In dem Auge des Patienten gibt es mehrere optische Grenzflächen zwischen den unterschiedlichen optischen Medien des Auges. Da sich an diesen Grenzflächen Brechungsindizes ändern, kommt es an den Grenzflächen zu einer Rückspiegelung der Lichtmuster. Im Rahmen der Erfindung werden diese Rückspiegelungen auch als "Testmarken" bezeichnet. Der Kern der Erfindung besteht nun darin, gleichzeitig oder sequentiell Testmarken bekannter Konturen und Positionen von verschiedenen optischen Grenzflächen im Auge zurückspiegeln und miteinander vergleichen zu können. Die wichtigsten Grenzflächen sind dabei die Oberfläche der Hornhaut (Cornea), sowie die Vorder- und die Rückseite der implantierten, künstlichen Linse. Weitere Grenzflächen wären die Rückseite der Cornea sowie die Netzhaut.

Bei der Erfindung weist die Abbildungsoptik mehrere Beobachtungsstrahlengänge aufweist, die unterschiedliche Abbildungseigenschaften auf, insbesondere unterschiedliche Fokussiereigenschaften. Dies macht es möglich, gleichzeitig die aus unterschiedlichen Tiefen des Augen reflektierten Lichtmuster scharf abzubilden, um sie miteinander vergleichen zu können.

Durch die Lagebeziehung der Lichtreflexe beziehungsweise Testmarken aus einer bestimmten Tiefe des Auges, insbesondere an der Vorder- oder Rückfläche der Intraokularlinse, kann auf die Ausrichtung der Linse zurückgeschlossen werden, insbesondere auf die Ausrichtung der Hauptachsen bei einer torischen Linse. Aus dem Vergleich der Lagebeziehung der Testmarken in einer bestimmten Tiefe des Auges und der Lagebeziehung der Testmarken auf der Oberfläche des Auges kann zudem ermittelt werden, welche Drehausrichtung die Intraokularlinse relativ zu einem Astigmatismus der Cornea hat. Dabei muss nicht unbedingt qualitativ ermittelt werden, wie die resultierende Gesamtbrechkraft oder Sehstärke des Auges ist. Es genügt vielmehr, dass aus den Lagebeziehungen der Lichtmuster zurückgeschlossen werden kann auf eine Drehung (unter Angabe der Drehrichtung und des Drehwinkels), durch die die in das Auge implantierte Linse optimal zum Astigmatimus der Cornea ausgerichtet werden kann.

Die Optikanordnung kann insbesondere so ausgebildet sein, dass sie als Gerät intra- oder postoperativ am menschlichen Auge oder als Laborgerät angewendet werden kann. Beispielsweise könnte die Optikanordnung als Optikmodul aufgebaut sein und zur intraoperativen Anwendung über entsprechende Halterungen unter ein OP-Mikroskop geschoben beziehungsweise geklappt werden oder in den Strahlengang eines OP-Mikroskops eingebracht werden. Alternativ könnte die Optikanordnung jedoch auch so aufgebaut sein, dass sie als eigenständiges Gerät benutzt werden kann.

Vorzugsweise sind die Kollimationsstrahlengänge jeweils nicht-kollinear zum Beobachtungsstrahlengang auf das Auge gerichtet, d. h. unter einem Winkel zum Beobachtungsstrahlengang. Dies führt zu einem seitlichen Versatz der Reflexionen der Lichtmuster aus unterschiedlichen Tiefen beziehungsweise von unterschiedlichen Grenzflächen des Auges, so dass die Testmarken beziehungsweise die Reflexionen der Lichtmuster besser erkennbar werden.

Als besonders geeignete Winkel zwischen dem Kollimationsstrahlengang und dem Beobachtungsstrahlengang haben sich Winkel von 10° bis 60° erwiesen, vorzugsweise im Bereich von 25° bis 40°. Bei diesen Winkeln entsteht ein gut erkennbarer, seitlicher Versatz der Testmarken, während das von den Testobjekten ausgehende Licht gleichzeitig gut auch in tiefere Regionen des Auges eindringt.

Besonders zweckmäßig ist es, wenn der Winkel zwischen dem Kollimationsstrahlengang und dem Beobachtungsstrahlengang variabel einstellbar ist. Auf diese Weise können der seitliche Versatz der Testmarken unter der Blickrichtung des Beobachters und das Eindringen der Lichtmuster in tiefere Regionen des Auges für den jeweiligen Anwendungsfall optimiert werden.

In einer zweckmäßigen Variante der Erfindung sind die Testobjekte gemeinsam um eine optische Achse des Beobachtungsstrahlengangs rotierbar. Dies ermöglicht es, auch unabhängig von der Ausrichtung des Patientenkopfes Testmarken aus unterschiedlichen Richtungen auf das Auge einstrahlen zu können. Aus der Veränderung der Testmarken (d. h. der Reflexionen der von den Testobjekten ausgehenden Lichtmuster) können dabei Rückschlüsse auf die Verläufe der Grenzflächen zwischen den optischen Medien des Auges gezogen werden, insbesondere über die Lage der Hauptachsen einer torischen IOL. Denkbar wäre es auch, dass solche Rückschlüsse aus der Veränderung einer Form einer Testmarke bei einer Rotation der Testobjekte um die optische Achse des Beobachtungsstrahlengangs gewonnen werden, beispielsweise aus einer Längenänderung einer linienförmigen Testmarke.

Die Licht ausstrahlenden Testobjekte können prinzipiell beliebige Konturen aufweisen. Als relativ einfache Konturen, die dennoch gut unterscheidbar sind, haben sich Linien, Doppellinien, Mehrfachlinien, Kreuze, Hohlkreuze, Doppelkreuze, Mehrfachkreuze, Dreiecke, Polygone, Pfeile, Buchstaben und/oder Symbole erwiesen.

Damit die Testobjekte Licht aussenden können, können sie selbstleuchtend sein, beispielsweise als Glühdrähte oder als LED-Felder. Alternativ können die Testobjekte jedoch auch reflektierend oder hinterleuchtet sein, Letzteres vorzugsweise als hinterleuchtete Blende mit bestimmter Kontur.

Als günstig hat es sich erwiesen, wenn sich jedes Testobjekt in seiner Farbe und/oder in seiner Kontur von einem anderen Testobjekt unterscheidet. Dies ermöglicht dem Bediener der Optikanordnung eine schnelle Zuordnung einzelner Lichtreflexe beziehungsweise Testmarken im Auge zu bestimmten Testobjekten. Eine farbliche Unterscheidung der Testobjekte ermöglicht auch dann eine eindeutige Identifizierung der einzelnen Testmarken, wenn diese unter einem bestimmten Beobachtungswinkel direkt hintereinander liegen sollten. Zusätzlich oder alternativ könnten die von den verschiedenen Testobjekten ausgehenden Lichtmuster auch unterschiedlich polarisiert werden. Mittels der unterschiedlichen Polarisationen könnten ebenfalls hintereinander liegende Testmarken aufgelöst, mittels polarisationsabhängiger Strahlteiler in unterschiedliche Beobachtungsstrahlengänge eingebracht und so gleichzeitig scharf abgebildet werden.

Die Handhabung der erfindungsgemäßen Optikanordnung kann weiter dadurch verbessert werden, dass zwei Testobjekte eine zueinander komplementäre Kontur aufweisen. Beispielsweise kann es sich um zwei aufeinander gerichtete Pfeile handeln, oder um ein Hohlkreuz (d. h. ein nicht leuchtendes, kreuzförmiges Feld) in Verbindung mit einer komplementären, kreuzförmigen Testmarke. Sobald die beiden komplementären Testmarken bei der Beobachtung des Auges eine bestimmte, sich ergänzende Position einnehmen, ist dadurch eine vorgegebene Ausrichtung mit der künstlichen Linse im Auge definiert, beispielsweise eine Ausrichtung der IOL, bei der die Hauptachsen mit den Hauptachsen des cornealen Astigmatismus übereinstimmen.

In einer weiteren, zweckmäßigen Variante der Erfindung ist für mindestens ein Testobjekt eine Beleuchtungseinrichtung mit einstellbarer Lichtstärke vorgesehen. Allgemein kann für jedes Testobjekt eine solche Beleuchtungseinrichtung mit einstellbarer Lichtstärke vorgesehen sein, insbesondere mit einer Einstellbarkeit der Lichtstärke unabhängig von der Lichtstärke der Beleuchtungseinrichtungen für andere Testobjekte. Dies erlaubt es, Unterschiede in der Lichtstärke der aus verschiedenen Tiefen des Auges reflektierten Testmarken ausgleichen zu können. Diese Unterschiede der Lichtstärke können sich ansonsten dadurch ergeben, dass die Lichtmuster unterschiedlich weite Wege im Auge zurücklegen müssen und dabei unterschiedlich viel Licht insbesondere durch Streuung verlieren. Die Möglichkeit, die Lichtstärken der Testmarken aus verschiedenen Tiefen aneinander angleichen zu können, erleichtert das Vergleichen der Lage der Testmarken aus verschiedenen Tiefen des Auges erheblich.

Weitere vorteilhafte Ausgestaltungen der Optikanordnung sind denkbar. So könnte zusätzlich im Beobachtungsstrahlengang ein Stabilisierungssystem gegen Bewegungen des Patientenauges vorgesehen sein, beispielsweise nach dem Bildverdopplungsprinzip. Prismen oder ähnlich optische Systeme könnten vorgesehen sein zur Bildumkehr von Testmarken, die bei der Abbildung eine Seitenumkehr erfahren. An einem Okular im Beobachtungsstrahlengang könnten eine oder mehrer Skalen vorteilhaft sein, die zum Beispiel die Winkel der Kollimationsstrahlengänge zum Beobachtungsstrahlengang, bestimmte Krümmungsradien der Cornea oder der IOL und/oder die Achslage einer IOL angeben.

Die Erfindung bezieht sich auch auf ein Verfahren zum Ermitteln der Ausrichtung einer künstlichen Linse in einem Auge. Bei diesem Verfahren werden zwei oder mehr Lichtmuster, die jeweils von einem Testobjekt mit definierter Kontur ausgehen und mittels je einer Kollimationsoptik kollimiert sind, unter zum Beobachtungsstrahlengang nicht-kollinearen Winkeln auf ein Auge gerichtet. Gleichzeitig werden die von der Hornhautvorderseite und/oder von mindestens einer Grenzfläche zwischen der künstlichen Linse und optischen Medien des Auges reflektierten Lichtmuster scharf abgebildet. Durch Verändern des Rotationswinkels der künstlichen Linse im Auge und/oder durch Verändern der Drehwinkel der Testobjekte relativ zum Auge können die Abbildungen der Reflexionen des oder der Lichtmuster von den unterschiedlichen Grenzflächen des Auges optimiert beziehungsweise maximiert werden. Bei maximaler Überlagerung der Reflexionen der Lichtmuster ist eine optimal Ausrichtung der künstlichen Linse im Auge gewährleistet. Bei einer torischen IOL bedeutet dies, dass ihre Hauptachsen exakt mit den Hauptachsen des Hornhaut-Astigmatismus des Auges übereinstimmen.

Erfindungsgemäß sorgen mehrere Beobachtungsstrahlengänge mit unterschiedlichen Abbildungseigenschaften dafür, dass die Reflexionen der Lichtmuster aus unterschiedlichen Tiefen des Auges, beispielsweise von der Hornhautvorderseite und/oder von mindestens einer Grenzfläche zwischen der künstlichen Linse und dem optischen Medien des Auges, gleichzeitig scharf abgebildet werden. Dies hat den Vorteil, dass die Ausrichtung der künstlichen Linse schneller erkannt werden kann als bei einem sequentiellen Abbilden der Reflexionen der Lichtmuster.

Besonders aussagekräftig wird das Verfahren, wenn der Winkel zwischen dem Kollimationsstrahlengang und dem Beobachtungsstrahlengang verändert wird und/oder wenn die Testobjekte gemeinsam um eine optische Achse des Beobachtungsstrahlengangs rotiert werden, um aus unterschiedlichen Richtungen Testmarken auf das Patientenauge projizieren zu können.

Weitere Varianten der erfindungsgemäßen Optikanordnung und des erfindungsgemäßen Verfahrens sind denkbar. Sie werden im Folgenden beschrieben.

Wie bereits vorstehend erläutert, ist es denkbar, die Optikanordnung oder das Verfahren so zu konfigurieren, dass eine Messung der Krümmungsradien der künstlichen Augenlinse möglich ist, nämlich unter Berücksichtung der Lage der von der Linse reflektierten Testmarken. Damit ist auch eine direkte Messung der Stärke der Linse möglich, ohne dazu eine Berechnung durchführen zu müssen. Insbesondere kann das aufwendige Messen und Auswerten von Wellenfronten vermieden werden. Vielmehr kann der Krümmungsradius direkt aus dem Abstand mehrerer Testmarken voneinander oder aus dem Abstand einer Testmarke von der optischen Achse abgelesen werden.

Hilfreich kann das Einkoppeln eines Licht-Zielstrahls, beispielsweise eines Laser-Zielstrahls, in die Optikanordnung sein, den der Patient während der Untersuchung fixiert. Damit trägt der Patient aktiv zu einer Lagestabilisierung seines Auges und damit zu einer erhöhten Präzision der Messung bei. Der Zielstrahl kann dabei so verlaufen, dass er zentral zu den auf das Auge projizierten Testmarken liegt. Dadurch wird eine zur Fixationsachse des Patientenauges zentrierte Ausrichtung erreicht. Die Einkopplung des Zielstrahls in die Optikanordnung kann am patientennahen Ende des Beobachtungsstrahlenganges erfolgen oder auch innerhalb des Beobachtungsstrahlenganges, beispielsweise mittels prismatischer Komponenten.

Besonders günstig ist es dabei, wenn der Zielstrahl nicht in Form eines winzigen Punktes, sondern als ausgedehntes Objekt auf das Auge gestrahlt wird. Beispielsweise (wenn auch keineswegs zwingend) könnte der Zielstrahl als rotationssymmetrische Fixationsmarke auf das Auge projiziert werden, beispielsweise als Hohlkreis, oder auch als T-förmige Markierung. Durch eine Überlagerung der Rückreflexe (zum Beispiel das Ineinanderlegen von kreisförmigen Lichtreflexen oder das Übereinanderlegen T-förmiger Reflexe) von Corneavorderfläche, Linsenvorder- beziehungsweise Linsenrückfläche kann eine Zentrierung der Intraokularlinse auf die vom Zielstrahl vorgegebene Fixationsachse erreicht werden.

Die Untersuchung des Auges wird erleichtert, wenn in mindestens einem der Beobachtungsstrahlengänge ein Bildverdopplungssystem angeordnet ist. Solch ein Bildverdopplungssystem ist dazu konfiguriert, das Bild des Auges in einer Zwischenbildebene zu verdoppeln, um so das für den Betrachter erzeugte Bild gegen Bewegungen des Auges zu stabilisieren.

In diesem Zusammenhang kann es vorteilhaft sein, wenn optische Elemente wie Prismen oder Linsen des Bildverdopplungssystems mit einer Beschichtung versehen sind, die auf die Lichtfrequenz der zum Projizieren der Testmarken auf das Auge und auf die künstliche Linse verwendeten Lichtquelle abgestimmt ist. Insbesondere könnte die Beschichtung dabei als schmalbandiger transmissiver Filter für das Licht der Testmarken ausgebildet sein. Durch diese wellenlängensensitive Beschichtung könnte folglich in einem Beobachtungsstrahlengang nur das Licht der Testmarke durchgelassen werden, sodass für den Bediener störende Doppelbilder vermieden werden. Die Beschichtung kann also einen spektral sehr schmalen Filter bilden, sodass in dem betreffenden Strahlengang nur das Reflexbild der Testmarke sehr hell zu sehen wäre, ein Doppelbild jedoch nur sehr schwach. Als schmalbandiger Filter kann die Beschichtung betrachtet werden, wenn sie nur Licht innerhalb eines Wellenlängenbereichs mit einer FWHM-Breite von etwa 10 nm ohne Abschwächung passieren lässt.

Durch das Vorhandensein mehrerer Beobachtungsstrahlengänge bietet sich eine weitere Verbesserung, wenn in mindestens einem der Beobachtungsstrahlengänge ein mechanisch ansteuerbarer, temporär nutzbarer Shutter vorgesehen ist, um diesen Beobachtungsstrahlengang temporär ausblenden zu können. Durch die zeitweise Ausblendung eines Beobachtungsstrahlengangs kann der Bediener (beziehungsweise Operateur) zwischen einem Operations-Modus ohne Doppelbilder und einem Mess-Modus mit Doppelbildern umschalten. Der Shutter kann an einem beliebigen Punkt eines der Beobachtungsstrahlengänge liegen.

Besonders robust kann ein Bildverdopplungssystems ausgebildet sein, wenn statt eines mehrteiligen Linsensystems durch Kombination mehrerer Prismen eine monolithische Ausführung des Bildverdopplungssystems erreicht wird. Dabei könnten auch die Prismen in der vorstehend beschriebenen Weise mit einer Beschichtung versehen sein.

Denkbar ist es ferner, die Optikanordnung inklusive der Beleuchtungs-, der Kollimations- und der Beobachtungselemente drehbar so unter einem binokularen Mikroskop anzuordnen, dass die Beobachtung über den einen Strahlengang des Binokular-Mikroskops erfolgt, während der zweite Strahlengang unbeeinflusst und insbesondere nicht abgedeckt ist. Sollten sich durch das Einfügen der Optikanordnung unterschiedliche optische Weglängen der beiden binokularen Mikroskopstrahlengänge ergeben, könnte dies durch geeignete optische Elemente wie zum Beispiel Prismen kompensiert werden.

Eine weitere Variante bestünde im Einbringen einer zusätzlichen Kollimations- und/oder Spiegeloptik in den Beobachtungsstrahlengang, die dazu ausgebildet ist, die durch die Krümmung der Linsenvorderfläche üblicherweise nach außen reflektierten Testmarken wieder in den Beobachtungsstrahlengang einzubringen und so für eine Messung verfügbar zu machen. Beispielsweise könnte in den Beobachtungsstrahlengang ein reflektierender Ring eingebracht werden, der die nach außen gerichteten Reflexe wieder in Richtung der optischen Achse des Beobachtungsstrahlengangs reflektiert, wo diese Reflexe über weitere Optikelemente wie Spiegel oder Prismen für eine Messung nutzbar gemacht werden.

Im Folgenden werden vorteilhafte Ausführungsbeispiele der erfindungsgemäßen Optikanordnung und des erfindungsgemäßen Verfahrens anhand einer Zeichnung näher erläutert. Im Einzelnen zeigen:
- Figur 1: eine schematische Darstellung der erfindungsgemäßen Optikanordnung,
- Figur 2: eine Darstellung der Reflexionen der eingestrahlten Lichtmuster bzw. der Testmarken am Auge,
- Figur 3a und 3b: einige Beispiele für Testmarken bzw. für die Konturen von Testobjekten ,
- Figur 4: eine Variante der Optikanordnung,
- Figur 5: eine zweite Variante eines Beobachtungsstrahlenganges,
- Figur 6: eine dritte Variante eines Beobachtungsstrahlenganges und
- Figur 7: eine Ansicht des Beobachtungsstrahlenganges aus der Perspektive eines Patienten.

Gleiche Komponenten sind in den Figuren durchgängig mit gleichen Bezugszeichen versehen.

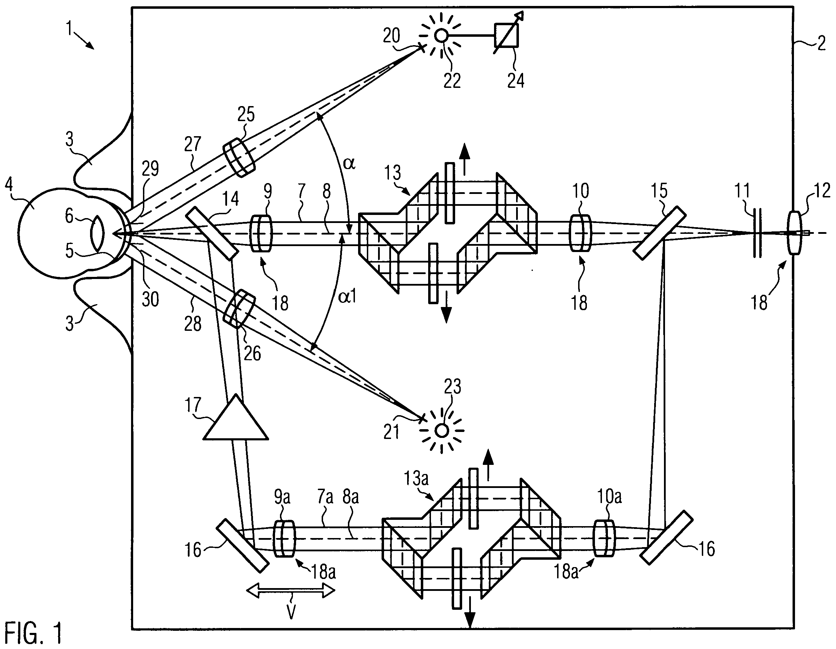

Figur 1 zeigt schematisch ein Ausführungsbeispiel einer erfindungsgemäßen Optikanordnung 1. Die Optikanordnung 1 verfügt über ein Gehäuse 2, mit dem die Optikanordnung 1 als eigenständiges Gerät konzipiert sein kann. Alternativ wäre es denkbar, dass das Gehäuse 2 Teil eines Operationsmikroskops ist oder durch entsprechende Halterungen in den Strahlengang eines Operationsmikroskops eingebracht werden kann, beispielsweise durch Hineinschwenken in den Strahlengang des OP-Mikroskops.

An dem Gehäuse 2 ist eine schematisch angedeutete Anlage 3 dargestellt, an die ein Patient seinen Kopf anlegen kann, um sein Auge 4 in eine vorgegebene Position relativ zum Gehäuse 2 und damit zur Optikanordnung 1 zu bringen. Die Anlage 3 erleichtert es zudem, das Patientenauge 4 möglichst lagestabil in der vorgegebenen Position zu halten.

Das Patientenauge 4 ist in Figur 1 ebenfalls lediglich schematisch dargestellt. Zu erkennen sind dabei die Hornhaut (Cornea) 5 des Auges, die zur Optikanordnung 1 ausgerichtet ist, sowie die Linse 6 des Auges. Bei der Linse 6 handelt es sich insbesondere um eine künstliche Augenlinse, auch bezeichnet als Intraokularlinse (IOL).

Die Optikanordnung 1 weist einen primären Beobachtungsstrahlengang 7 auf. Die zentrale, optische Achse 8 des primären Beobachtungsstrahlengangs 7 definiert die Richtung, unter der das Auge 4 beobachtbar ist. In dem Beobachtungsstrahlengang 7 sind ein erstes Objektiv 9 und ein zweites Objektiv 10 vorgesehen. Sie dienen dazu, in einer Bildebene 11 ein Bild des beobachteten Bereichs des Auges 4 zu erzeugen. Mittels eines Okulars 12 oder zweier nebeneinander angeordneter Okulare 12 wird dieses in der Bildebene 11 erzeugte Bild scharf für den Betrachter abgebildet. Zwischen den beiden Objektiven 9, 10 ist ein Bildverdopplungssystem 13 angeordnet. Es dient dazu, das Bild des Auges 4 in einer Zwischenbildebene zu verdoppeln, um das für den Betrachter erzeugte Bild gegen Bewegungen des Auges 4 zu stabilisieren.

Die Optikanordnung 1 verfügt ferner über einen zweiten oder sekundären Beobachtungsstrahlengang 7a. Über einen ersten Stahlteiler 14, der zwischen dem Auge 4 und dem ersten Objektiv 9 angeordnet ist, ist der sekundäre Beobachtungsstrahlengang 7a aus dem primären Beobachtungsstrahlengang 7 ausgekoppelt. Über einen zweiten Strahlteiler 15, der zwischen dem zweiten Objektiv 10 und der Bildebene 11 angeordnet ist, wird der sekundäre Beobachtungsstrahlengang 7a wieder in den primären Beobachtungsstrahlengang 7 eingekoppelt, sodass das Auge 4 für den durch das Okular 12 blickenden Betrachter gleichzeitig über beide Beobachtungsstrahlengänge 7, 7a beobachtbar ist. Umlenkspiel 16 führen den sekundären Beobachtungsstrahlengang 7a von der Auskopplung aus dem primären Strahlengang 7 am ersten Strahlteiler 14 zu der Einkopplung in den primären Strahlengang 7 vom zweiten Strahlteiler 15.

Ebenso wie im primären Strahlengang 7 sind auch im sekundären Beobachtungsstrahlengang 7a ein erstes Objektiv 9a und ein zweites Objektiv 10a vorgesehen, um ein Bild des beobachteten Bereichs des Auges 4 zu erzeugen und dieses Bild am Okular 12 scharf für den Betrachter abzubilden. Ebenfalls in Analogie zum primären Beobachtungsstrahlengang 7 ist auch im sekundären Beobachtungsstrahlengang 7a ein Bildverdopplungssystem 13a eingebaut, um das erzeugte Bild gegen Bewegungen des Auges 4 zu stabilisieren. Ein Unterschied zum primären Beobachtungsstrahlengang 7 besteht nun jedoch darin, dass das erste Objektiv 9a des sekundären Beobachtungsstrahlengangs 7a nicht in einer festen Position angeordnet ist, sondern entlang der optischen Achse 8a des sekundären Beobachtungsstrahlengang 7a verschoben werden kann. Diese Verschiebebewegung ist angedeutet durch einen Doppelpfeil V. Die Verschiebebewegung V kann beispielsweise über einen Bereich von bis zu 10, bis zu 20 oder auch bis zu 30 mm ermöglicht sein. Sie erlaubt es, Bilder aus unterschiedlichen Tiefen im Auge 4 sequentiell scharf für den Betrachter abzubilden. Ein weiterer Unterschied zum primären Beobachtungsstrahlengang 7 besteht darin, dass im sekundären Beobachtungsstrahlengang 7a ein Bildumkehrmittel 17 vorgesehen ist, das im vorliegenden Ausführungsbeispiel durch ein Prisma realisiert ist. Es dient dazu, ein eventuell auf dem Kopf stehendes Bild im sekundären Beobachtungsstrahlengang 7a umzukehren.

Die beiden Objektive 9, 10 und das Okular 12 bilden zusammen eine Abbildungsoptik 18 des primären Beobachtungsstrahlengangs 7, die die Abbildungseigenschaften dieses primären Beobachtungsstrahlengang 7 festlegt. Analog bilden das erste Objektiv 9a, das zweite Objektiv 10a und wiederum das Okular 12 die Abbildungsoptik 18a des zweiten Beobachtungsstrahlengangs 7a, die dessen Abbildungseigenschaften definieren. Durch die Möglichkeit zum Verschieben des ersten Objektivs 9a sind die Abbildungseigenschaften des sekundären Beobachtungsstrahlengang 7a variabel bzw. einstellbar.

Die erfindungsgemäße Optikanordnung 1 umfasst des Weiteren ein erstes Testobjekt 20 und ein zweites Testobjekt 21. Die beiden Testobjekte 20, 21 haben jeweils eine definierte, vorzugsweise eine zueinander komplementäre Kontur. Von den Testobjekten 20, 21 geht in der Form dieser definierten Kontur Licht aus. Zu diesem Zweck können die Testobjekte 20, 21 selbstleuchtend sein, beispielsweise in Form eines LED-Feldes oder selbstleuchtender Folien (OLED). Im dargestellten Ausführungsbeispiel hingegen handelt es sich bei den Testobjekten 20, 21 jeweils um mit der definierten Kontur versehene Blenden, die von einer Beleuchtungseinrichtung 22, 23 hinterleuchtet sind. Die Beleuchtungseinrichtungen 22, 23 können dabei entweder die selbe Farbe haben, beispielsweise in Form von Weißlicht, oder sie könnten zu einer Unterscheidbarkeit der beiden Testobjekte 20, 21 auch unterschiedliche Farben haben, insbesondere Komplementärfarben wie grün und rot, oder unterschiedliche Polarisationen. Im dargestellten Ausführungsbeispiel ist die Beleuchtungseinrichtung 22 für das erste Testobjekt 20 mit einem Regler 24 versehen, mittels dessen die Lichtstärke dieser Beleuchtungseinrichtung 22 stufenlos oder in bestimmten Stufen regelbar ist.

Jedem Testobjekt 20, 21 ist jeweils eine eigene Kollimationsoptik 25, 26 zugeordnet. Diese Kollimationsoptik 25, 26 kollimiert das von dem jeweiligen Testobjekt 20, 21 ausgehende, konturierte Lichtmuster und bündelt es in einen zum Auge 4 gerichteten Kollimationsstrahlengang 27, 28. Das von den Testobjekten 20 bzw. 21 ausgehende Lichtmuster gelangt damit als paralleles Strahlenbündel, das aus dem Unendlichen zu kommen scheint, auf das Auge 4. Dort wird das Licht an den Grenzflächen zwischen zwei optischen Medien mit unterschiedlichem Berechungsindex reflektiert, insbesondere an der Vorderseite oder der Rückseite der Hornhaut 5 sowie an der Vorderseite oder der Rückseite der künstlichen Linse 6; letztere Grenzfläche wirkt dabei als Hohlspiegel. Die Reflexion bzw. der reflektierte Lichtfleck wird im Rahmen der Erfindung auch als Testmarke 29, 30 bezeichnet. Diese Reflexionen bzw. Testmarken 29, 30 werden über die beiden Beobachtungsstrahlengänge 7, 7a für den Beobachter scharf dargestellt.

Die Kollimationsstrahlengänge 27, 28 befinden sich in der Optikanordnung 1 jeweils unter einem Winkel α, α1 zur optischen Achse 8 des primären Beobachtungsstrahlengangs 7. Die beiden Winkel α, α1 können dabei gleich sein. In einer vorteilhaften Ausführungsform sind die Lage der Testobjekte 20, 21 und die Ausrichtung der Kollimationsstrahlengänge 27, 28 hinsichtlich der zwischen den Kollimationsstrahlengängen 27, 28 und den Beobachtungsstrahlengängen 7 eingenommenen Winkel α, α1 veränderbar. Eine geeignete Mechanik kann dafür sorgen, dass die Winkel α, α1 einander dabei stets gleich bleiben. In der Nähe des Okulars 12 kann eine Skala angebracht sein, die die jeweiligen Winkel α für den Benutzer anzeigt. Alternativ könnte dieser Winkel auch mit einer Skala in das Okular 12 eingeblendet werden. Darüber hinaus sind alle Testobjekte 20, 21 vorzugsweise gemeinsam um die optische Achse 8 des Beobachtungsstrahlengangs 7 rotierbar, um die Testmarken 29, 30 aus unterschiedlichen Richtungen auf das Auge 4 einstrahlen zu können.

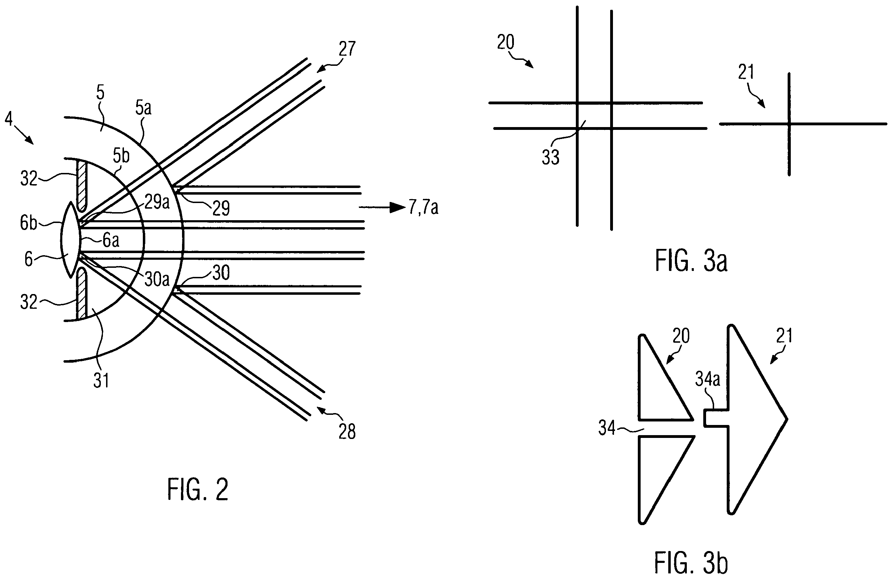

Figur 2 zeigt in vergrößerter Darstellung die Situation am Patientenauge 4. Dabei sind (nicht maßstabsgerecht) lediglich die vorderen Medien des Auges 4 dargestellt, nämlich die Hornhaut 5, die (künstliche) Linse 6 sowie die dazwischen liegende Vorderkammer 31 und die Iris 32. An der Vorderseite 5a und der Rückseite 5b der Hornhaut 5 sowie an der Vorderseite 6a und der Rückseite 6b der Linse 6 finden jeweils Brechungsindexsprünge zwischen den verschiedenen optischen Medien des Auges 4 bzw. zwischen der Hornhaut 5 und der Luft statt. An diesen Grenzflächen 5a, 5b, 6a, 6b kommt es aufgrund der Berechungsindexsprünge zu Rückspiegelungen der über die Kollimationsstrahlengänge 27, 28 eingestrahlten Lichtmuster der Testobjekte 20, 21. In Figur 2 ist deutlich zu erkennen, dass die Reflexionen 29, 29a, 30, 30a dieses Lichtmuster, die über die Beobachtungsstrahlengänge 7, 7a für den Betrachter als helle "Testmarken" sichtbar werden, aus unterschiedlichen Tiefen im Auge stammen. Die Reflexionen bzw. Testmarken 29, 30 stammen dabei von der Vorderseite 5a der Hornhaut 5, während Testmarken 29a, 30a von der Vorderseite 6a der künstlichen Linse 6 stammen, die sich ca. 5 bis 10 mm unterhalb der Hornhautvorderfläche 5a befinden. Weitere Reflexionen bzw. Testmarken könnten von der Rückseite 6b der künstlichen Linse 6 reflektiert werden, die dort als Hohlspiegel wirkt.

Figur 3a zeigt exemplarisch die Konturen zweier gemeinsam verwendeter Testobjekte 20, 21. Dabei besteht die Kontur des ersten Testobjekts 20 aus einem Vierfachkreuz, d.h. aus einer Gruppe von zwei parallelen Linien, die im rechten Winkel von einer Gruppe zweier anderer, ebenfalls paralleler Linien geschnitten wird. Im Zentrum der Kontur dieses Testobjekts 20 entsteht dabei ein rechteckiger, insbesondere quadratischer Zentrumsbereich 33. Bei der Kontur des zweiten Testobjekts 20 handelt es sich um ein einfaches Kreuz. Die Konturen der beiden Testobjekte 20, 21 sind insofern komplementär zueinander, als die kreuzförmige Kontur des zweiten Testobjekts 21 so ausgerichtet werden kann, dass sich das Zentrum dieses Kreuzes mittig im zentralen Bereich 33 des Lichtmusters des ersten Testobjekts 20 befindet.

Figur 3b zeigt ein zweites Ausführungsbeispiel der Konturen der beiden Testmarken 20, 21. Die links dargestellte, erste Testmarke 20 hat dabei die Kontur eines gleichschenkligen Dreiecks, dem ein mittlerer Streifen 34 fehlt. Das zweite Testobjekt 21 hat die Kontur eines ebensolchen gleichschenkligen Dreiecks. Hier ist jedoch der Mittelstreifen vorhanden und sogar in Form eines Vorsprungs 34a über das Dreieck hinaus verlängert. Die beiden Testmarken 20, 21 sind insofern komplementär zueinander, als der verlängerte, vorstehende Abschnitt 34a der zweiten Testmarke 21 in den ausgesparten Mittelstreifenbereich 34 der ersten Testmarke 20 in hineingebracht werden kann.

Im Folgenden wird nun der Betrieb der erfindungsgemäßen Optikanordnung bzw. der Ablauf des erfindungsgemäßen Verfahrens exemplarisch beschrieben.

Zunächst wird der Kopf des Patienten der Optikanordnung 1 angedockt, insbesondere durch Anlegen des Patientenkopfes an die Anlage 3. Dies erfolgt, nachdem eine künstliche Augenlinse 6, ggf. eine torische Augenlinse 6, in das Patientenauge 4 eingesetzt worden ist. Diese Linse 6 ist zuvor speziell für genau dieses Patientenauge 4 gefertigt worden, um Fehlsichtigkeiten des Auges 4, insbesondere einen Hornhautastigmatismus, zu kompensieren. Herstellermarkierungen auf der künstlichen Linse 6 markieren die Lage der Hauptachsen, wenn es sich um eine torische IOL handelt. Diese Markierungen sind für einen Beobachter sichtbar, der durch das Okular 12 blickt, und das Auge 4 durch den Beobachtungsstrahlengang 7 beobachtet. Die Markierungen sind jedoch nicht immer völlig korrekt mit den Hauptachsen der IOL ausgerichtet.

An dieser Stelle kommt nun das erfindungsgemäße Verfahren zum Tragen. Ähnlich wie bei einem Keratometer werden die von den Testobjekten 20, 21 ausgehenden Lichtmuster definierter Kontur auf das Auge 4 eingestrahlt. Die Reflexionen dieser Lichtmuster sind in Form von hellen Testmarken für den Beobachter sichtbar. Sie stammen jedoch, wie vorstehend erwähnt und insbesondere anhand von Figur 2 erläutert, aus unterschiedlichen Tiefen des Auges. Um diese Tiefenunterschiede zu kompensieren, kann nun neben dem primären Beobachtungsstrahlengang 7 ein sekundärer Beobachtungsstrahlengang 7a vorgesehen sein, dessen Abbildungsoptik 18 aus einer anderen Tiefe des Auges 4 ein scharfes Bild erzeugt als die Abbildungsoptik 18 des primären Beobachtungsstrahlengangs 7. Auf diese Weise werden die Testmarken aus unterschiedlichen Tiefen des Auges 4 für den Betrachter gleichzeitig scharf abgebildet. Wenn dabei Helligkeitsunterschiede zwischen den Testmarken bestehen und insbesondere die aus größerer Tiefe im Auge 4 reflektierten Testmarken 29a, 30a lichtschwächer sein sollten als die aus höheren Regionen des Auges 4 reflektierten Testmarken, können die Helligkeitsunterschiede durch eine Regelung der Lichtstärke der Beleuchtungseinrichtung 22 kompensiert werden. Alternativ ist es denkbar, dass im sekundären Beobachtungsstrahlengang 7a oder auch im primären Beobachtungsstrahlengang 7, vor allem wenn nur ein Beobachtungsstrahlengang 7 vorhanden ist, die Abbildungseigenschaften der Abbildungseigenschaften der Abbildungsoptik 18 veränderbar sind, insbesondere durch Verfahren eines Objektivs 9, 9a. Dies ermöglicht es dem Betrachter, sequentiell die Testmarken scharf abzubilden, die von der Hornhautvorderfläche 5a oder von Grenzflächen in tieferen Regionen des Auges 4 reflektiert werden, ohne dass dazu der Betrachtungswinkel geändert werden müsste.

In einem weiteren Schritt kann der Betrachter nun entweder die Ausrichtung der Testobjekte 20, 21 relativ zum Auge 4 verändern, vorzugsweise unter Beibehaltung des Abstands zwischen den Testmarken 20, 21 und dem Auge 4, oder er kann die gesamte Gruppe aller Testobjekte 20, 21 um die optische Achse 8 des primären Beobachtungsstrahlengangs 7 rotieren. Auf diese Weise werden die Lichtmuster der Testobjekte 20, 21 aus unterschiedlichen Richtungen auf das Auge 4 gerichtet. Diese Richtungsänderung wiederum ermöglicht es, die von den Testobjekten 20, 21 hervorgerufenen Reflexionen bzw. Testmarken übereinander zu bringen. Erleichtert wird dies durch Unterschiede in den Konturen der Testmarken 20, 21, die eine Unterscheidung der beiden Testmarken für den Betrachter ermöglichen. Noch weiter erleichtert wird das Maximieren der Überlagerung der Testmarken dadurch, dass den Testobjekten 20 einander komplementäre Konturen gegeben werden, wie dies in den Figuren 3a, 3b exemplarisch gezeigt ist. Zudem kann der Betrachter bedarfsweise die künstliche Linse 6 im Auge 4 rotieren, um die Überlagerung der Testmarken zu maximieren. Eine optimale Ausrichtung der künstlichen Linse 6 im Auge 4 erkennt der Betrachter daran, dass die Überlagerung sowohl der von der Hornhautvorderfläche 5a, als auch der von einer Grenzfläche 6a, 6b der Linse 6 reflektierten Lichtmuster maximiert ist.

Ausgehend von dem dargestellten Ausführungsbeispiel können die erfindungsgemäße Optikanordnung 1 und das erfindungsgemäße Verfahren in vielfacher Weise verändert oder erweitert werden. Denkbar wäre es beispielsweise, dass nicht nur zwei Testobjekte 20, 21, sondern vier, sechs oder eine beliebige andere Anzahl von Testobjekten 20, 21 vorgesehen ist. Zudem wäre es denkbar in den Beobachtungsstrahlengängen 7, 7a Kameras mit einer geeigneten Bildauswertungssoftware einzubauen. Zusätzlich zur einer Rotierbarkeit um die optische Achse 8 des Beobachtungsstrahlengangs 7 könnten die Testobjekte 20, 21 auch translatorisch bewegbar sein.

Dadurch, dass die Bilder der sich überlagernden oder nebeneinander liegenden Testmarken 29, 30 aus verschiedenen Tiefen im Augen 4 gleichzeitig oder sequentiell in eine gemeinsame Abbildungsebene gebracht werden, können die rückgespiegelten Markierungen mehrerer optischer Grenzflächen 5a, 6a, 6b miteinander verglichen werden. Dadurch ist es z.B. möglich, die Achse der Zylinderanteile der Cornea 5 und einer torischen IOL 6 zu überlagern und sie auf Achsübereinstimmung zu prüfen. Intra- oder postoperativ kann damit eine entsprechende Korrektur der Rotation oder Positionierung der IOL 6 vorgenommen werden. Dabei erfolgt keine Berechnung der Stärke der IOL 6, sondern vielmehr eine direkte Messung der Stärke und der Position der Linse 6 über ihre Krümmungsradien. Fehlausrichtungen, die sich aus fehlerhaft angebrachten Herstellermarkierungen auf der Linse 6 ergeben könnten, werden bei der Erfindung vermieden. Damit werden die Brechungseigenschaften des Auges 4 insgesamt verbessert. Im Gegensatz zu herkömmlichen Systemen, die auf dem Vergleich von präoperativ und intraoperativ gewonnenen Bildern des Patientenauges 4 beruhen, entfallen bei der Erfindung zudem Fehler durch Rotationsabweichungen zwischen den präoperativen und intraoperativen Bildern. Bei der vorliegenden Erfindung wird vielmehr die Lage der IOL 6 und der Cornea 5 relativ zueinander unabhängig von einer möglichen Drehung des Patientenauges 4 oder des gesamten Patientenkopfes gemessen.

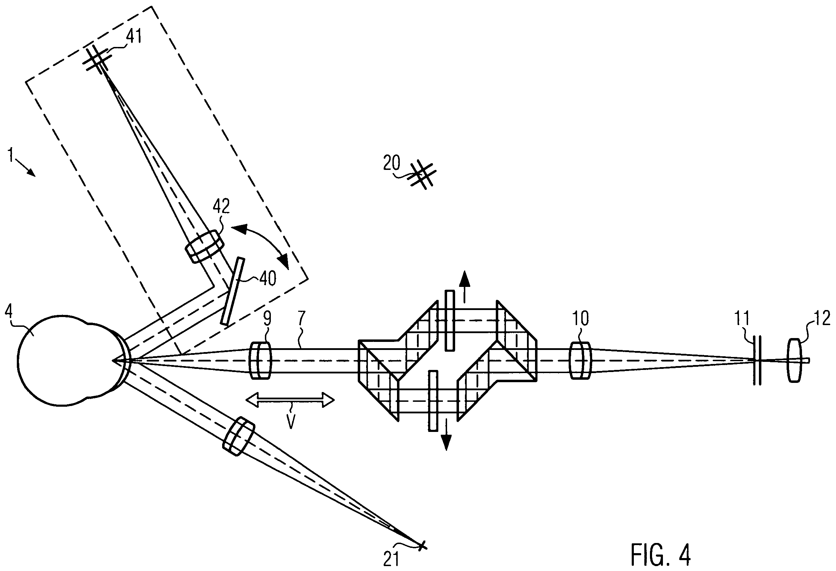

Eine weitere Variante der Optikanordnung 1 mit erweiterter Funktionalität ist in Figur 4 gezeigt. Der Übersichtlichkeit halber zeigt Figur 4 eine Variante der Optikanordnung 1 mit lediglich einem einzigen Beobachtungsstrahlengang 7, womit diese Variante als solche nicht erfindungsgemäß ist. Um Reflexionen der Testmarken 29, 30 aus unterschiedlichen Tiefen des Auges 4 scharf abbilden zu können, ist das Objektiv 9 in diesem Beobachtungsstrahlengang 7 verschiebbar, wie durch den Pfeil V angegeben.

Die Änderung gegenüber dem ersten Ausführungsbeispiel besteht darin, dass die Optikanordnung 1 durch die Funktionalität eines Retinoskops ergänzt ist. Dies geschieht dadurch, dass ein schwenkbarer Stahlteiler oder Spiegel 40 in den Kollimationsstrahlengang 27 zwischen dem ersten Testobjekt 20 und dem Auge 4 eingebracht werden kann. Durch das Einschwenken dieses Spiegels 40 wird der Kollimationsstrahlengang unterbrochen. Stattdessen gelangt nun das Licht von einem Spalt 41 über eine Kollimationsoptik 42 auf das Auge 4. Dadurch wird auf dem Auge 4 ein Lichtspalt erzeugt, der durch eine Bewegung der Spaltblende 41 senkrecht zu der optischen Achse des von ihr ausgestrahlten Lichts verfahren werden kann.

Der Lichtspalt wird kollimiert über das zu untersuchende Auge 4 verfahren und der Reflex an der Retina durch das Okular 12 betrachtet. Die Richtung der Reflexbewegung bei Bewegung über den Bereich der Pupille wird mit der ursprünglichen Bewegungsrichtung des Spaltes verglichen:
- Keine Bewegung des Reflexes: das Auge hat keine Fehlsichtigkeit,
- Mitläufige Bewegung: das Auge ist hyperop (weitsichtig),
- Gegenläufige Bewegung: das Auge ist myop (kurzsichtig).

Die Änderung der Bewegungsrichtungen kann auch bei Stabsichtigkeit (Astigmatismus) des Auges 4 genutzt werden, hierfür können die Achsen mit dem größten und kleinsten Brechwert durch Drehen des Lichtspaltes und Verfahren über die Pupille bestimmt werden. Entsprechend kann auch die Fehlsichtigkeit direkt nach Implantation einer (astigmatismuskorrigierenden, torischen) Kunstlinse 6 unter dem OP-Mikroskop bestimmt werden, ggf. durch einen Vergleich vor und nach Implantation der Kunstlinse.

Hierfür ist eine Vorrichtung vorteilhaft, die den Lichtspalt an das Mikroskop ankoppelt, so dass der Reflex direkt durch das Okular 12 des Mikroskops betrachtet werden kann. Die Vorrichtung muss für eine Bestimmung der Achslage drehbar sein und der Lichtspalt durch Verschieben in einer horizontalen Richtung über die Pupille des Patientenauges 4 bewegt werden können. Um einen Vergleich verschiedener Positionen der drehbaren Ankopplung vornehmen zu können, ist eine Markierung im Okular einstellbar (drehbar) angebracht.

In dieser Variante umfasst die Optikanordnung 1 eine Vorrichtung zur drehbaren Ankopplung einer Projektionseinheit bestehend aus einer Lichtquelle (Halogenlampe, LED, ...), einem Spalt, einer Kollimationsoptik, einem Strahlteiler (insgesamt als Aufbau nach dem Prinzip des Retinoskops) und einer (horizontalen) Verschiebeeinrichtung z.B. durch ein über Kugellager verbundenes Ringsystem unterhalb oder innerhalb eines OP-Mikrokopes. Die Verschiebung in Richtung der kurzen Spaltachse kann z.B. über eine Schraubvorrichtung oder eine elektromotorische Verfahrung erreicht werden. Die Dreheinrichtung ist mindestens von 0 bis 180° drehbar, eine Drehung bis 360 Grad ist möglich. Der Verschiebebereich des Lichtspaltes auf Pupillenebene des Patientenauges 4 beträgt 0 - 25 mm. Der Lichtspalt hat eine Abmessung von 1 - 25 mm Länge und 0.1 - 10 mm Breite.

Optional lässt sich mindestens eine der Testmarken zu einem Spalt umwandeln, z.B. durch Einschieben eines Spaltes als Projektions "blende" oder bei einer kreuzförmigen Lichtquelle durch Verkleinern der Dimension des Bildes in einer Richtung (zusammenfahren eines Balkens des Kreuzes), und über einen Strahlteiler verschiebbar kollinear in den Strahlengang der OP-Mikroskops einblenden/einschieben/einschwenken.

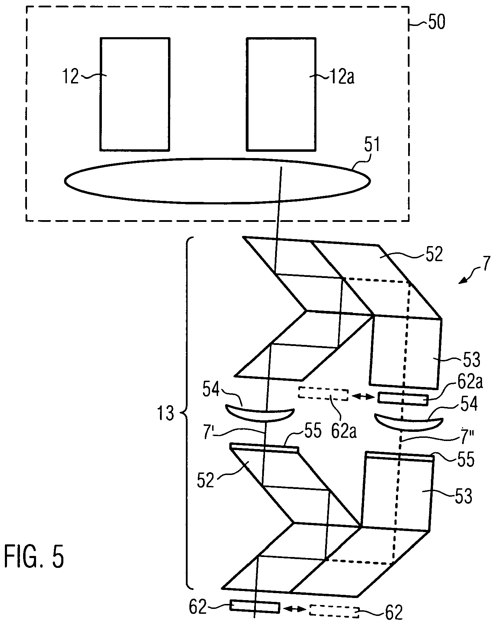

Figur 5 zeigt schematisch eine weitere Variante, bei der die Optikanordnung an ein OP-Mikroskop 50 angekoppelt ist, insbesondere ein Binokular-Mikroskop. Dargestellt sind die beiden Okulare 12, 12a des Binokular-Mikroskops sowie eine Linse, die das Hauptobjektiv 51 des OP-Mikroskops repräsentiert. Von der Optikanordnung 1 ist der Übersichtilichkeit halber lediglich ein Teil des Beobachtungsstrahlengangs 7 inklusive des Bildverdopplungssystems 13 dargestellt.

Zu erkennen ist, dass das Bildverdopplungssystem 1 lediglich im Strahlengang des einen Okulars 12a des Mikroskops 50 angeordnet ist, sodass der Strahlengang des anderen Okulars 12 nicht beeinflusst ist. Zu sehen ist ferner, dass das Bildverdopplungssystem 13 eine Vielzahl von miteinander verbundenen Prismen 52 und transparenten optischen Blöcken 53 aufweist. Diese Prismen 52 und optischen Blöcke 53 sind so miteinander verbunden, dass sich eine Strahlteileranordnung ergibt, die den Beobachtungsstrahlengang 7 in zwei Teilstrahlen 7' und 7" aufteilt und diese Teilstrahlen anschließend wieder miteinander überlagert. In jedem der Teilstrahlengänge 7', 7" befindet sich eine Freistrahlstrecke, in der dezentriert eine Linse 54 plaziert ist. Die dezentrierte Anordnung der Linsen 54 bewirkt eine Strahlablenkung.

Die Optikanordnung inklusive des Beobachtungsstrahlenganges 7 ist vorzugsweise drehbar an das OP-Mikroskop gekoppelt. Denkbar ist es, den Einfluss des Bildverdopplungssystems 13 auf die optische Weglänge im Beobachtungsstrahlengang des Okulars 12a durch eine entsprechende Kompensationsoptik im Strahlengang des anderen Okulars 12 zu kompensieren, beispielsweise unter Verwendung von Prismen.

Denkbar ist es ferner, ein oder mehrere der optischen Elemente 52, 53, 54 des Bildverdopplungssystems 13 mit einer Beschichtung 55 zu versehen, die hier lediglich beispielhaft auf einem Prisma 52 und einem transparenten optischen Block 53 dargestellt ist. Bei der Beschichtung 55 kann es sich um einen schmalbandigen transmissiven Filter für das von den Testobjekten 20 beziehungsweise von den Testmarken 29, 30 ausgestrahlte Licht handeln.

Ferner zeigt Figur 5 exemplarisch ein weiteres Merkmal, das in allen Ausführungsbeispielen der Optikanordnung 1 vorhanden sein kann, nämlich einen ansteuerbaren Shutter 62. Dieser kann temporär in die mit durchgezogenen Linien dargestellte Position im Beobachtungsstrahlengang 7 eingebracht werden, um diesen Beobachtungsstrahlengang 7 temporär auszublenden. Ansonsten nimmt der Shutter 62 eine geöffnete Position ein, die mit gestrichelten Linien dargestellt ist.

Zusätzlich oder alternativ kann ein ansteuerbarer Shutter 62a auch innerhalb eines Bildverdopplungssystems 13 angeordnet sein - und zwar so, dass er in einer mit durchgezogenen Linien gezeigten Position nur den einen Teilstrahl 7" blockiert, während er in der geöffneten Stellung (mit gestrichelten Linien gezeigt) diesen Teilstrahl 7" nicht behindert. Wenn sich der Shutter 62a im Teilstrahlengang 7" befindet und diesen ausblendet, werden Doppelbilder verhindert, weil der Beobachter nur das Licht sieht, das durch den anderen Teilstrahlengang 7' in das Okular 12a gelangt.

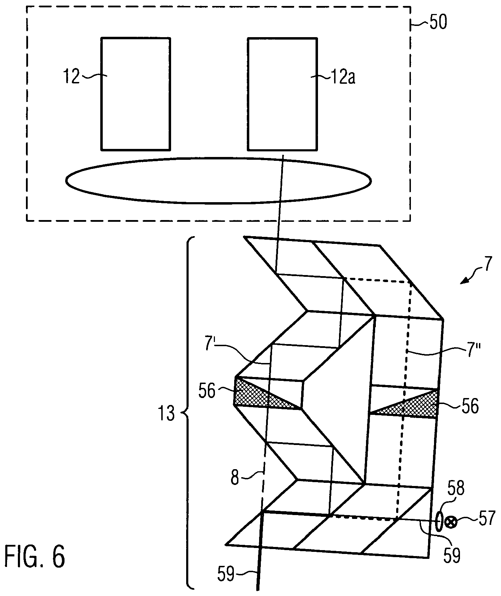

Figur 6 zeigt eine weitere Abwandlung der in den Figuren 1, 4 und 5 gezeigten Bildverdopplungssysteme 13. Analog zu Figur 5 ist auch bei der Situation in Figur 6 die Optikanordnung mit dem dargestellten Bildverdopplungssystem 13 an ein OP-Mikroskop 50 angekoppelt, insbesondere lediglich in den Strahlengang eines der beiden Okulare 12a des Binokular-Mikroskops 50 eingefügt. Im Unterschied zur Situation in Figur 5 ist das Bildverdopplungssystem 13 gemäß Figur 6 nun als monolithisches System ausgebildet, das ohne Freistrahlstrecken auskommt. Anstelle der Freistrahlstrecken mit den dezentrierten Linsen 54 sind Prismendubletts 56 in jedem der Teilstrahlengänge 7, 7', 7" angeordnet, die für eine Netto-Strahlablenkung der beiden Teilstrahlen sorgen.

Figur 6 zeigt noch eine weitere Variation, die in allen Ausführungsbeispielen der Optikanordnung 1 vorgesehen sein kann, nämlich ein Fixierlicht 57. Bei der Lichtquelle für dieses Fixierlicht 57 kann es sich um eine LED, eine Glühlampe oder einen Laser handeln. Mittels einer Kollimationslinse 58 wird der von dem Fixierlicht 57 ausgestrahlte Zielstrahl 59 in das Bildverdopplungssystem 13 und damit in den Beobachtungsstrahlengang 7 der Optikanordnung 1 eingekoppelt. Diese Einkopplung erfolgt so, dass der Zielstrahl 59 parallel zur optischen Achse 8 in den Beobachtungsstrahlengang 7 gelenkt wird. Während einer Untersuchung seines Auges 4 soll und kann ein Patient den Zielstrahl 59 fixieren, um sein Auge 4 so optimal zur optischen Achse 8 des Beobachtungsstrahles 7 auszurichten.

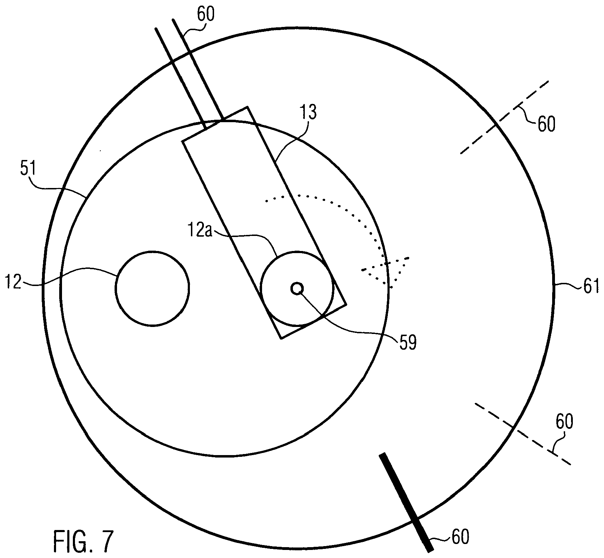

Figur 7 zeigt schematisch die Ansicht des Beobachtungsstrahlenganges 7 aus Sicht eines Patienten. Im Zentrum des Blickfeldes des Patienten befindet sich der Zielstrahl 59, der vom Patienten fixiert werden soll. Er befindet sich seinerseits im Zentrum des Bereichs, der durch das Okular 12a des Binokular-Mikroskops 50 beobachtbar ist. Das Bildverdopplungssystem 13 ist so angeordnet, dass es den Beobachtungsweg des zweiten Okulars 12 nicht behindert. Ausgehend von der in Figur 7 gezeigten Stellung kann das Bildverdopplungssystem 13 in der durch den gestrichelten Pfeil angegebenen Richtung um weit mehr als 180°, sogar um etwa 270° gedreht werden, ohne den Beobachtungsweg des zweiten Okulars 12 zu befinden. Am Rand des Gesichtsfeldes können sich Ophthalmometer-Marken 60 und ein zusätzlicher Leuchtkreis 61 befinden, die die Orientierung erleichtern.

In einer weiteren Variation könnte die erfindungsgemäße Optikanordnung beziehungsweise das erfindungsgemäße Verfahren dazu geeignet sein, auch die axiale Lage einer Intraokularlinse 6 im Auge zu messen, d. h. die Lage der Linse 6 in der sogenannten z-Achse. Möglich ist dies, wenn ein bekannter Krümmungsradius sowie eine bekannte Dicke der Intraokularlinse 6 vorausgesetzt werden. Die Messung der axialen Lage der Intraokularlinse 6 kann ebenfalls aus der Größe der zurückgespiegelten Testmarken beziehungsweise aus dem Abstand verschiedener Testmarken voneinander beziehungsweise aus dem Abstand einer Testmarke von der optischen Achse ermittelt werden. Mit dieser Information über die axiale Lage der Intraokularlinse 6 kann die refaktive Wirkung der Linse 6 exakt berechnet werden.

## Patentansprüche

1. Optikanordnung (1) zum Ermitteln der Ausrichtung einer künstlichen Linse (6) in einem Auge (4), umfassend mindestens einen Beobachtungsstrahlengang (7, 7a) mit einer Abbildungsoptik (18, 18a), mindestens zwei Licht ausstrahlende Testobjekte (20, 21) mit definierter Kontur, sowie pro Testobjekt (20, 21) eine ihm zugeordnete Kollimationsoptik (25, 26) zum Kollimieren des von dem Testobjekt (20, 21) ausgehenden Lichtmusters in einem auf das Auge (4) gerichteten Kollimationsstrahlengang (27, 28),
wobei mehrere auf ein gemeinsames Okular (12) führende Beobachtungsstrahlengänge (7, 7a) mit unterschiedlichen Abbildungseigenschaften vorgesehen sind, die derart eingestellt sind, dass gleichzeitig die von verschiedenen Grenzflächen reflektierten Lichtmuster (29, 30) abbildbar sind,
wobei die Grenzflächen die Hornhautvorderseite und mindestens eine der Grenzflächen zwischen der künstlichen Linse (6) und optischen Medien des Auges (4) umfassen.

2. Optikanordnung nach Anspruch 1, **dadurch gekennzeichnet, dass** der Kollimationsstrahlengang (27, 28) jeweils nicht-kollinear zum Beobachtungsstrahlengang (7, 7a) auf das Auge (4) gerichtet ist.

3. Optikanordnung nach einem der vorangehenden Ansprüche, **dadurch gekennzeichnet, dass** der Winkel (α, α1) zwischen dem Kollimationsstrahlengang (27, 28) und dem Beobachtungsstrahlengang (7, 7a) von 10° bis 60° beträgt, vorzugsweise von 25° bis 40°.

4. Optikanordnung nach einem der vorangehenden Ansprüche, **dadurch gekennzeichnet, dass** die Testobjekte (20, 21) gemeinsam um die optische Achse (8) rotierbar sind, welche die Richtung definiert, unter der das Auge (4) beobachtbar ist.

5. Optikanordnung nach einem der vorangehenden Ansprüche, **dadurch gekennzeichnet, dass** ein Testobjekt (20, 21) selbstleuchtend, hinterleuchtet oder reflektierend ist, wobei sich vorzugsweise jedes Testobjekt (20) in seiner Farbe und/oder Kontur von einem anderen Testobjekt (21) unterscheidet.

6. Optikanordnung nach einem der vorangehenden Ansprüche, **dadurch gekennzeichnet, dass** zwei Testobjekte (20, 21) eine zueinander komplementäre Kontur aufweisen.

7. Optikanordnung nach einem der vorangehenden Ansprüche, **dadurch gekennzeichnet, dass** die Optikanordnung (1) zum Messen der Krümmungsradien einer künstlichen Linse (6) konfiguriert ist.

8. Optikanordnung nach einem der vorangehenden Ansprüche, **dadurch gekennzeichnet, dass** ein Zielstrahl zum Fixieren durch den Patienten in die Optikanordnung (1) eingekoppelt ist.

9. Optikanordnung nach einem der vorangehenden Ansprüche, **dadurch gekennzeichnet, dass** in mindestens einem der Beobachtungsstrahlengänge (7, 7a) ein Bildverdopplungssystem (13, 13a) angeordnet ist.

10. Optikanordnung nach Anspruch 9, **dadurch gekennzeichnet, dass** mindestens ein optisches Element des Bildverdopplungssystems (13, 13a) mit einer Beschichtung (55) versehen ist, die als transmissiver Filter für des von dem Testobjekten (20, 21) ausgehende Licht ausgebildet sind.

11. Optikanordnung nach einem der vorangehenden Ansprüche, **dadurch gekennzeichnet, dass** in mindestens einem der Beobachtungsstrahlengänge (7, 7a) und/oder in einem Teilstrahlengang (7', 7") eines Bildverdopplungssystems (13) ein ansteuerbarer Shutter (62, 62a) vorgesehen ist.

12. Optikanordnung nach einem der Ansprüchen 9 bis 11, **dadurch gekennzeichnet, dass** das Bildverdopplungssystem (13, 13a) durch Kombination mehrerer Prismen (52) monolithisch ausgebildet ist.

13. Optikanordnung nach einem der vorangehenden Ansprüche, **dadurch gekennzeichnet, dass** die Optikanordnung (1) drehbar an einem binokularen Mikroskop (50) befestigt ist.

14. Verfahren zum Ermitteln der Ausrichtung einer künstlichen Linse (6) in einem Auge (4), mit folgenden Schritten:
- Beobachten des Auges (4) über mehrere Beobachtungsstrahlengänge (7, 7a), wobei mittels mehrerer auf ein gemeinsames Okular (12) führender Beobachtungsstrahlengänge (7, 7a) mit unterschiedlichen Abbildungseigenschaften gleichzeitig die Reflexionen der Lichtmuster von den verschiedenen Grenzflächen abgebildet werden,
- Einstrahlen von zwei oder mehr Lichtmustern, die jeweils von einem Testobjekt (20, 21) mit definierter Kontur ausgehen und mittels je einer Kollimationsoptik (25, 26) kollimiert sind, unter zum Beobachtungsstrahlengang (7) nicht-kollinearen Winkeln auf das Auge (4),
- gleichzeitiges Abbilden der von verschiedenen Grenzflächen reflektierten Lichtmuster (29, 30), wobei die Grenzflächen die Hornhautvorderseite und mindestens eine Grenzfläche zwischen der künstlichen Linse (6) und optischen Medien des Auges (4) umfassen,
- Verändern des Rotationswinkel der künstlichen Linse (6) im Auge (4) und/oder der Drehwinkel der Testobjekte (20, 21) um die optische Achse (8) eines der Beobachtungsstrahlengänge, welche die Richtung definiert, unter der das Auge (4) beobachtbar ist, zum Maximieren einer Überlagerung der Abbildung der Reflexionen der Lichtmuster von den verschiedenen Grenzflächen.

15. Verfahren nach Anspruch 14, **dadurch gekennzeichnet, dass** ein Zielstrahl (59) zum Fixieren durch einen Patienten in die zum Durchführen des Verfahrens verwendete Optikanordnung (1) eingekoppelt wird.

16. Verfahren nach einem der Ansprüche 14 oder 15, **dadurch gekennzeichnet, dass** mittels eines in mindestens einem Beobachtungsstrahlengang (7, 7a) angeordneten Bildverdopplungssystems (13, 13a) eine Stabilisierung eines für den Betrachter erzeugten Bildes gegen Bewegungen des Auges (4) erfolgt.

17. Verfahren nach einem der Ansprüche 14 bis 16, **dadurch gekennzeichnet, dass** einer der Beobachtungsstrahlengänge (7, 7a) und/oder ein Teilstrahlengang (7', 7") in einem Bildverdopplungssystem (13) temporär mittels eines ansteuerbaren Shutters (62, 62a) ausgeblendet wird.

18. Verfahren nach einem der Ansprüche 14 bis 17, **dadurch gekennzeichnet, dass** eine Zentrierung der Intraokularlinse (6) über eine zentralsymmetrische Anordnung der Rückreflexe der verschiedenen Grenzflächen geprüft wird.

19. Verfahren nach einem der Ansprüche 14 bis 18, **dadurch gekennzeichnet, dass** mittels einer Größenmessung und/oder eines Größenvergleichs der reflektierten Testmarken der verschiedenen Grenzflächen eine Bestimmung der Position der Intraokularlinse (6) in axialer Richtung entlang der optischen Achse erfolgt.

## Claims

1. Optical arrangement (1) for determining the orientation of an artificial lens (6) in an eye (4), comprising at least one observation beam path (7, 7a) with an imaging optical system (18, 18a), at least two light-emitting test objects (20, 21) with a defined contour, and one collimation optical system (25, 26) per test object (20, 21) associated with it for collimating the light pattern emanating from the test object (20, 21) into a collimation beam path (27, 28) directed at the eye (4),
wherein multiple observation beam paths (7, 7a) are provided leading to a common ocular (12) and having different imaging properties which are configured such that the light patterns (29, 30) reflected by different boundaries are simultaneously imaged, wherein the boundaries comprise the front side of the cornea and at least one boundary between the artificial lens (6) and optical media of the eye (4).

2. Optical arrangement according to claim 1, **characterized in that** the collimation beam path (27, 28), respectively, is directed at the eye (4) in a manner non-collinear to the observation beam path (7, 7a).

3. Optical arrangement according to one of the preceding claims, **characterized in that** the angle (α,α1) between the collimation beam path (27, 28) and the observation beam path (7, 7a) is from 10° to 60°, preferably from 25° to 40°.

4. Optical arrangement according to one of the preceding claims, **characterized in that** the test objects (20, 21) are jointly rotatable about the optical axis (8) which defines the direction under which the eye (4) is observable.

5. Optical arrangement according to one of the preceding claims, **characterized in that** a test object (20, 21) is self-luminous, back-lit or reflecting, wherein preferably each test object (20) differs from another test object (21) in its color and/or contour.

6. Optical arrangement according to one of the preceding claims, **characterized in that** two test objects (20, 21) have mutually complementary contours.

7. Optical arrangement according to one of the preceding claims, **characterized in that** the optical arrangement (1) is configured for measuring the radii of curvature of an artificial lens (6).

8. Optical arrangement according to one of the preceding claims, **characterized in that** a pilot beam is coupled into the optical arrangement (1) to be fixed by the patient.

9. Optical arrangement according to one of the preceding claims, **characterized in that** in at least one of the observation beam paths (7, 7a), an image reduplication system (13, 13a) is arranged.

10. Optical arrangement according to claim 9, **characterized in that** at least one optical element of the image reduplication system (13, 13a) is provided with a coating (55) which is embodied as transmissive filter for the light emanating from the test objects (20, 21).

11. Optical arrangement according to one of the preceding claims, **characterized in that** in at least one of the observation beam paths (7, 7a) and/or in a partial beam path (7', 7") of an image reduplication system (13), a controllable shutter (62, 62a) is provided.

12. Optical arrangement according to one of claims 9 to 11, **characterized in that** the image reduplication system (13, 13a) is designed monolithically by combination of multiple prisms (52).

13. Optical arrangement according to one of the preceding claims, **characterized in that** the optical arrangement (1) is rotatably mounted to a binocular microscope (50).

14. Method for determining the orientation of an artificial lens (6) in an eye (4), comprising the following steps:
- observing the eye (4) via a plurality of observation beam paths (7, 7a), wherein the reflections of light patterns from different boundaries are imaged simultaneously by means of a plurality of observation beam paths (7, 7a) leading to a common ocular (12) and having different imaging properties, respectively,
- irradiating one or two light patterns, which each emanate from a test object (20, 21) with a defined contour and are collimated by means of one collimation optical system (25, 26) each, onto the eye (4) at angles that are non-collinear with the observation beam path (7),
- simultaneous imaging of the light patterns (29, 30) reflected by different boundaries, these boundaries comprising the front side of the cornea and at least one boundary between the artificial lens (6) and optical media of the eye (4),
- changing the rotational angle of the artificial lens (6) in the eye (4) and/or the angles of rotation of the test objects (20, 21) about the optical axis (8) of one of the observation beam paths (7, 7a) defining the direction under which the eye (4) is observable for maximizing a superimposition of the image of the reflections of the light patterns from the different boundaries.

15. Method according to claim 14, **characterized in that** a pilot beam (59) to be fixed by a patient is coupled into an optical arrangement (1) for carrying out the method.

16. Method according to one of claims 14 or 15, **characterized in that** by means of an image reduplication system (13, 13a) arranged in at least one observation beam path (7, 7a), a stabilization of an image generated for the viewer against movements of the eye (4) is accomplished.

17. Method according to one of claims 14 to 16, **characterized in that** one of the observation beam paths (7, 7a) and/or a partial beam path (7', 7") is temporarily blocked in an image reduplication system (13) by means of a controllable shutter (62, 62a).

18. Method according to one of claims 14 to 17, **characterized in that** a centering of the intraocular lens (6) is checked via a central-symmetrical arrangement of the retroreflections of the different boundaries.

19. Method according to one of claims 14 to 18, **characterized in that** by means of size measurement and/or size comparison of the reflected test targets of the different boundaries, a determination of the position of the intraocular lens (6) in the axial direction along the optical axis is effected.

## Revendications

1. Agencement optique (1) pour déterminer l'orientation d'une lentille artificielle (6) dans un oeil (4), comprenant au moins un faisceau de rayons optiques d'observation (7, 7a) avec une optique de reproduction d'image (18, 18a), au moins deux objets de test (20, 21) émettant de la lumière, avec un contour défini, ainsi que, par objet de test (20, 21), une optique de collimation (25, 26), qui lui est associée, pour collimater la configuration de lumière émise par l'objet de test (20, 21) dans un faisceau de rayons optiques de collimation (27, 28) dirigé sur l'oeil (4), agencement dans lequel sont prévus plusieurs faisceaux de rayons optiques d'observation (7, 7a) menant à un oculaire commun (12), avec des propriétés de reproduction d'image différentes, qui sont réglés de manière à pouvoir reproduire simultanément les configurations de lumière (29, 30) réfléchies par des surfaces limites différentes, et
dans lequel les surfaces limites comprennent le côté avant de la cornée et au moins une des surfaces limites entre la lentille artificielle (6) et des milieux optiques de l'oeil (4).

2. Agencement optique selon la revendication 1, **caractérisé en ce que** le faisceau de rayons optiques de collimation (27, 28) est dirigé sur l'oeil (4), respectivement de manière non colinéaire au faisceau de rayons optiques d'observation (7, 7a).

3. Agencement optique selon l'une des revendications précédentes, **caractérisé en ce que** l'angle (α, α1) entre le faisceau de rayons optiques de collimation (27, 28) et le faisceau de rayons optiques d'observation (7, 7a) vaut de 10° à 60°, de préférence de 25° à 40°.

4. Agencement optique selon l'une des revendications précédentes, **caractérisé en ce que** les objets de test (20, 21) peuvent tourner en commun autour de l'axe optique (8), qui définit la direction sous laquelle l'oeil (4) peut être observé.

5. Agencement optique selon l'une des revendications précédentes, **caractérisé en ce qu'**un objet de test (20, 21) est luminescent, rétroéclairé ou réfléchissant, chaque objet de test (20) se distinguant de préférence dans sa couleur et/ou son contour, d'un autre objet de test (21).

6. Agencement optique selon l'une des revendications précédentes, **caractérisé en ce que** deux objets de test (20, 21) présentent un contour mutuellement complémentaire.

7. Agencement optique selon l'une des revendications précédentes, **caractérisé en ce que** l'agencement optique (1) est configuré pour mesurer les rayons de courbure d'une lentille artificielle (6).

8. Agencement optique selon l'une des revendications précédentes, **caractérisé en ce qu'**un rayon-cible destiné à être fixé par le patient, est envoyé dans l'agencement optique (1).

9. Agencement optique selon l'une des revendications précédentes, **caractérisé en ce que** dans l'un au moins des faisceaux de rayons optiques d'observation (7, 7a) est agencé un système de dédoublement d'image (13, 13a).

10. Agencement optique selon la revendication 9, **caractérisé en ce qu'**au moins un élément optique du système de dédoublement d'image (13, 13a) est muni d'un revêtement (55), qui est réalisé en tant que filtre transmissif pour la lumière émise par les objets de test (20, 21).

11. Agencement optique selon l'une des revendications précédentes, **caractérisé en ce que** dans l'un au moins des faisceaux de rayons optiques d'observation (7, 7a) et/ou dans un faisceau de rayons optiques partiel (7', 7") d'un système de dédoublement d'image (13), il est prévu un obturateur (62, 62a) pouvant être commandé.

12. Agencement optique selon l'une des revendications 9 à 11, **caractérisé en ce que** le système de dédoublement d'image (13, 13a) est réalisé de manière monolithique par la combinaison de plusieurs prismes (52).

13. Agencement optique selon l'une des revendications précédentes, **caractérisé en ce que** l'agencement optique (1) est fixé de manière rotative sur un microscope binoculaire (50).

14. Procédé pour déterminer l'orientation d'une lentille artificielle (6) dans un oeil (4), comprenant les étapes suivantes :
- l'observation de l'oeil (4) par l'intermédiaire de plusieurs faisceaux de rayons optiques d'observation (7, 7a), les réflexions des configurations de lumière des différentes surfaces limites étant reproduites simultanément au moyen de plusieurs faisceaux de rayons optiques d'observation (7, 7a) à propriétés de reproduction d'image différentes, menant à un oculaire (12) commun,
- l'envoi de deux configurations de lumière ou davantage, qui sont émises respectivement d'un objet de test (20, 21) à contour défini, et qui sont collimatées chacune respectivement par une optique de collimation (25, 26), sur l'oeil (4), sous des angles non colinéaires au faisceau de rayons optiques d'observation (7),
- la reproduction simultanée des configurations de lumière (29, 30) réfléchies par différentes surfaces limites, les surfaces limites comprenant le côté avant de la cornée et au moins une surface limite entre la lentille artificielle (6) et des milieux optiques de l'oeil (4),
- la modification de l'angle de rotation de la lentille artificielle (6) dans l'oeil (4) et/ou de l'angle de rotation des objets de test (20, 21) autour de l'axe optique (8) de l'un des faisceaux de rayons optiques d'observation, qui définit la direction sous laquelle l'oeil (4) peut être observé,
en vue de maximiser une superposition de la reproduction des réflexions des configurations de lumière des différentes surfaces limites.

15. Procédé selon la revendication 14, **caractérisé en ce qu'**un rayon-cible (59) destiné à être fixé par le patient, est envoyé dans l'agencement optique (1) utilisé pour la mise en oeuvre du procédé.

16. Procédé selon l'une des revendications 14 ou 15, **caractérisé en ce qu'**au moyen d'un système de dédoublement d'image (13, 13a) agencé dans au moins un faisceau de rayons optiques d'observation (7, 7a), on effectue une stabilisation d'une image produite pour l'observateur, à l'encontre de mouvements de l'oeil (4).

17. Procédé selon l'une des revendications 14 à 16, **caractérisé en ce que** l'un des faisceaux de rayons optiques d'observation (7, 7a) et/ou un faisceau de rayons optiques partiel (7', 7") dans un système de dédoublement d'image (13) est masqué temporairement au moyen d'un obturateur (62, 62a) pouvant être commandé.

18. Procédé selon l'une des revendications 14 à 17, **caractérisé en ce que** l'on vérifie un centrage de la lentille intraoculaire (6), par l'intermédiaire d'un agencement symétrique central des réflexions de retour des différentes surfaces limites.

19. Procédé selon l'une des revendications 14 à 18, **caractérisé en ce qu'**au moyen d'une mesure de grandeur et/ou d'une comparaison de grandeur des marques de test réfléchies des différentes surfaces limites, on effectue une détermination de la position de la lentille intraoculaire (6) dans la direction axiale le long de l'axe optique.
